# EUROPÄISCHE PATENTSCHRIFT

(11) **EP 1 201 633 B1**
(45) Veröffentlichungstag und Bekanntmachung des Hinweises auf die Patenterteilung: **23.06.2004**
(21) Anmeldenummer: 00123092.9
(22) Anmeldetag: 25.10.2000
(51) Int. Cl.: C07C 29/10, C07C 45/64, C07C 47/19, C07C 29/141, C07C 31/20, C07C 29/88

(54) **Verfahren zur Herstellung von 1,3-Propandiol**
Process for the production of 1,3-propanediol
Procédé pour la fabrication de 1,3 propanediol

(43) Veröffentlichungstag der Anmeldung: 02.05.2002
(73) Patentinhaber: E.I. DUPONT DE NEMOURS AND COMPANY, Wilmington, DE 19808 (US)
(72) Erfinder: Hahm, Torsten, Dr., 63584 Gründau-Rothenbergen (DE); Haas, Thomas, Dr., 60316 Frankfurt a. M. (DE); Ronge, Christian, 5033 Buchs im Aargau (CH)
(74) Vertreter: Morf, Jan Stefan, Dr. Dipl.-Chem.

(56) Entgegenhaltungen:
- EP-A- 0 577 972
- EP-A- 0 915 075
- CH-A- 502 971
- C.A. ROHJAN, ET AL.: "Über Polyäther des Trimethylenglykols" BERICHTE DER DEUTSCHEN CHEMISCHEN GESELLSCHAFT, Bd. 54, Nr. 11, 10. Dezember 1921 (1921-12-10), Seiten 3118-3121, XP002164675 Verlag Chemie, Weinheim, DE

## Beschreibung

Die Erfindung betrifft ein Verfahren zur Herstellung von 1,3-Propandiol(PDO)auf der Basis der Hydratisierung von Acrolein zu 3-Hydroxypropionaldehyd (HPA) mit anschließender katalytischer Hydrierung.

Erfindungsgemäß wird aus dem aus diesem Prozeß als Nebenprodukt entstehenden 4-Oxa-1,7-heptandiol (DiPDO) gleichfalls 1,3-Propandiol gewonnen.

Wie aus EP 0 577 972 bekannt ist, basieren die von Acrolein ausgehenden Verfahren zur Herstellung von 1,3-Propandiol auf zwei Reaktionsstufen, nämlich Stufe (a) der Hydratisierung von Acrolein in Gegenwart eines sauren Hydratisierungskatalysators, Stufe (b) der katalytischen Hydrierung des 3-Hydroxypropionaldehyd erhaltenden, von nicht umgesetztem Acrolein befreiten Reaktionsgemisches der Stufe (a) und Stufe (c), die destillative Aufarbeitung des Reaktionsgemisches.

Durch destillative Aufarbeitung des Reaktionsgemisches in der Stufe (c), wie Wassereindampfung, Restwasserdestillation, Zwischensiederdestillation und Reindestillation, wird reines 1,3-Propandiol gewonnen.

Ein Nachteil des bekannten Verfahrens zur Herstellung von 1,3-Propandiol besteht darin, daß durch verschiedene Nebenreaktionen, insbesondere während der Hydratisierungsstufe, die Gesamtausbeute an 1,3-Propandiol gemindert wird. Bei der Aufarbeitung des Reaktionsgemisches aus der katalytischen Hydrierung konnten in dem Hochsiederanteil (Siedepunkt oberhalb desjenigen von 1,3-Propandiol) als Hauptprodukte 4-Oxa-1,7-heptandiol (DiPDO) und 4-Hydroxy-3-hydroxymethyl-tetrahydropyran (H-HMT1 und H-HMT2) nachgewiesen werden.

Aufgabe der vorliegenden Erfindung ist, einen Weg aufzuzeigen, die Ausbeute an 1,3 Propandiol im Verfahren zur Herstellung von 1,3-Propandiol aus Acrolein in einfacher Weise zu erhöhen.

Gegenstand der Erfindung ist ein Verfahren zur Herstellung von 1,3-Propandiol, umfassend die Stufen
a) Hydratisierung von Acrolein in Gegenwart eines sauren Hydratisierungskatalysators,
b) katalytische Hydrierung des 3-Hydroxypropionaldehyd enthaltenden, von nicht umgesetztem Acrolein befreiten Reaktionsgemisch der Stufe (a) und
c) destillative Aufarbeitung des Wassers, 1,3-Propandiol und oberhalb 1,3-Propandiol siedende Nebenprodukte enthaltenden Reaktionsgemischs der Stufe (b),
welches dadurch gekennzeichnet ist, daß man den Hochsiedersumpf der Aufarbeitung (c) - auch als PDO-Sumpflösung bezeichnet -zwecks Etherspaltung in wäßriger Lösung in Gegenwart von Säure, wie zum Beispiel Schwefelsäure, im Temperaturbereich von 200°C bis 300°C, zum Beispiel in einem Tantalrohr, behandelt, die erhaltene Lösung neutralisiert und in die destillative Aufarbeitungsstufe (c) zurückführt.

Etherspaltungen können in an sich bekannter Weise in wäßriger Lösung in Gegenwart von Mineralsäuren durchgeführt werden. Allgemein kommen Mineralsäuren wie H₂SO₄, H₃PO₄ oder HNO₃ zum Einsatz. Auch organische Säuren wie Propionsäure (PrS), Trifluoressigsäure (F₃C-COOH) oder Pyridinhydrochlorid können für die Etherspaltung eingesetzt werden. Ether, wie beispielsweise 1-Phenoxynaphthalen und 9-Phenoxyphenanthren, sind durch sogenannte Aquathermolyse im Rohr aus V4A-Stahl nur in Gegenwart von Wasser in 1-Naphthol beziehungsweise 9-Hydroxyphenanthren und Phenol spaltbar.

Generell lassen sich Ether auch in der Gasphase spalten, wie zum Beispiel Spaltung von n-Butylalkylether beziehungsweise n-Butylarylether in Buten und Alkohole beziehungsweise Phenole oder Spaltung von Ester, Vinylether und Alkene mit Beta-ständigem Chlor im Rohrreaktor aus Vycor-Glas in ungesättigte Chlorverbindungen wie Vinylchlorid. Weiteres Beispiel ist die Gasphasenpyrolyse von Benzylphenylether im Glasbehälter in Gegenwart von Tetralin.

Auch in der Flüssigphase sind Etherspaltungen möglich, beispielsweise die Pyrolyse von Dibutylether im Reaktor aus Gold in n-Butan, Butyraldehyd und auch 1-Butanol.

Für die Spaltung der Ether werden desweiteren unter- und überkritische Lösungsmittel eingesetzt. So erhält man bei der Thermolyse von Benzylphenylether in unter- und überkritischem Wasser und überkritischem Methanol unter anderem Phenol und Toluol als Spaltprodukte.

Die in der vorliegenden Erfindung durchgeführte Etherspaltung kann bevorzugt im Tantalrohr mit Schwefelsäure durchgeführt werden.

In EP 0 577 972 Patent werden folgende Aussagen gemacht:
- Abtrennung von DiPDO ist notwendig

Der Vorteil unseres Verfahrens ist, daß der im Verfahren anfallende Hochsiedersumpf direkt eingesetzt werden kann. Es entstehen nicht, wie behauptet, Nebenprodukte, die nicht abzutrennen sind.
◆ Nachteil Feststoffkatalysatoren: kurze Standzeit
◆ Mineralsäurekatalysatoren sind nicht unzweckmäßig. Die Säure kann durch einen Ionentauscher entfernt werden. Ionentauscher können regeneriert werden. Oder es wird ein unlösliches Salz (im Fall von H₂SO₄) durch Zugabe von Kalkmilch Ca(OH)₂ gebildet, welches dann abfiltriert wird.

Eine Spaltlösung kann zusammen mit dem Roh-PDO Strom der Anlage problemlos in der vorhandenen Aufarbeitung ohne Modifikation destilliert werden. Es tritt kein Qualitätsverlust auf.

### Beispiele:

In einer Laborapparatur werden die Versuche zur DiPDO-Spaltung (Spaltung von 4-Oxa-1,7-Heptandiol) kontinuierlich durchgeführt. Die Apparatur besteht aus einer Vorlage für die mit organischer Säure beziehungsweise mit Mineralsäure versetzten wäßrigen DiPDO-Lösung, einer HPLC-Pumpe zur Förderung, einem GC-Ofen, in dem das Reaktionsrohr installiert ist. Für die Versuche mit Propionsäure wird ein Rohr aus V4A-Stahl (750 * 0,3 cm im Durchmesser) und für die Versuche mit Schwefelsäure ein Rohr aus Tantal (58 * 0,3 cm im Durchmesser)eingesetzt. Nach dem Reaktor wird die Reaktionslösung mittels Wasserkühlung auf Raumtemperatur gekühlt. Die Apparatur wird auf einem Druck von ca. 100 bar gehalten. Die Produktlösung wird in zeitlichen Abständen mittels GC (Flächen %) analysiert.

### 1) Spaltung von reinem DiPDO mit Propionsäure im Rohrreaktor aus V4A-Stahl

Versuchsbedingungen sind in Tabelle 1 und Analysenergebnisse in GC-Flächen % in Tabelle 2 dargestellt.

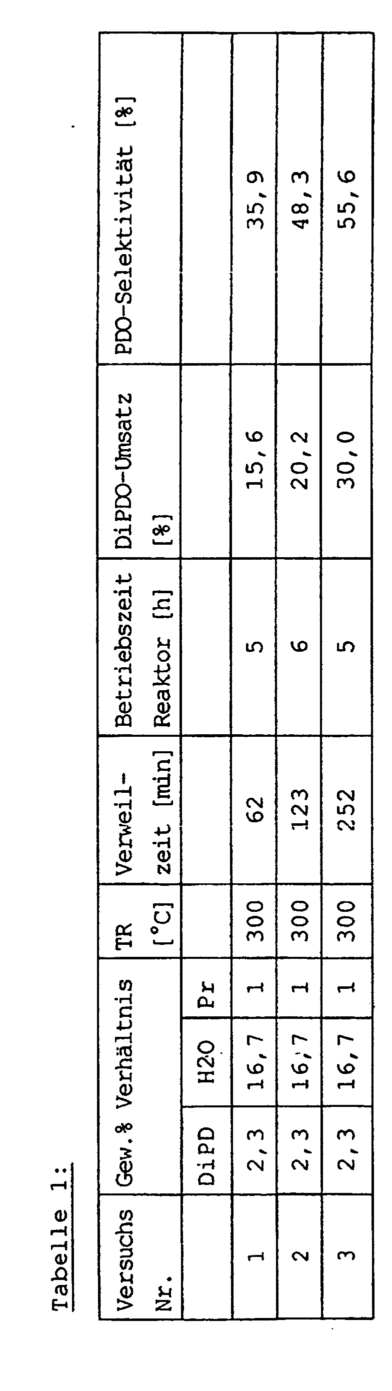

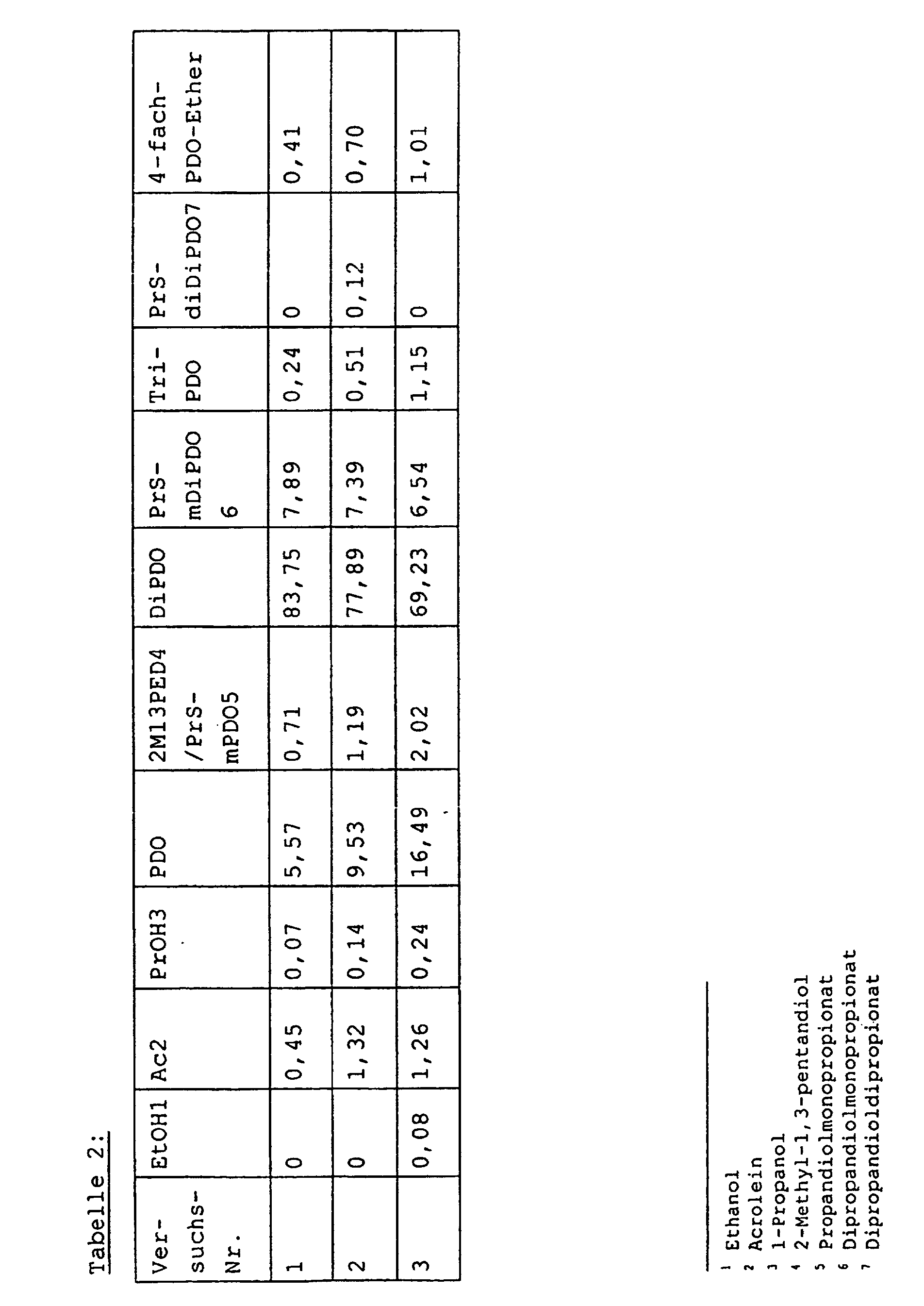

Mit steigender Verweilzeit nehmen Umsatz und Selektivität zu. Allerdings steigen hierbei auch die Nebenprodukte wie Ac, PrOH, PrS-mPDO, TriPDO sowie 4-fach-PDO-Ether an, wohingegen die PrS-mDiPDO-Bildung zurückgeht. Bei Erhöhung der Reaktionstemperatur auf 320°C und gleicher Verweilzeit (123 min) steigt der Umsatz von 20,2 % auf 32,3 % und die Selektivität von 48,3 % auf 54,6 %. Diese Werte erhält man auch mit niedrigerer Temperatur und doppelter Verweilzeit. Nachteil der Propionsäure ist die Ausbildung von Estern mit DiPDO als auch mit PDO.

### 2) Spaltung von reinem DiPDO mit Schwefelsäure im Tantalrohr

Bei diesen Versuchen werden das Gew.%-Verhältnis DiPDO:H₂O, die H₂SO₄-Konzentration (diese immer bezogen auf die Organik), die Temperatur sowie die Verweilzeit variiert.

Eine hohe DiPDO-Konzentration (Gew.%-Verhältnis DiPDO:H₂O von 1:1) führt zwar zu hohen Umsätzen (bis zu 87 %), allerdings beträgt die Selektivität nur 20 %. Es bilden sich neben TriPDO verstärkt 4-fach- und 5-fach-PDO-Polyether aus, welches sich optisch als ölige Flecken auf den Lösungen bemerkbar macht. Ist die DiPDO-Konzentration dagegen relativ gering (Gew.%-Verhältnis DiPDO:H20 von 1:10), liegen Umsatz (61 %) und Selektivität (73 %) zwar verhältnismäßig hoch, dafür ist aber der Ac-Gehalt mit ca. 4 % der höchste von allen durchgeführten Versuchen. Bei einer sehr niedrigen H₂SO₄-Konzentration von nur 0,05 % wird nur wenig DiPDO umgesetzt: 19 % bei 280°C.

Die Tabellen 3 und 4 zeigen die erhaltenen DiPDO-Umsätze und PDO-Selektivitäten mit den Einstellungen DiPDO:H₂O von 1:4 und 1:8 bei den Temperaturen 250°C und 280°C.

**Tabelle 3:**

| T = 250°C | | | T = 280°C | | |
|---|---|---|---|---|---|
| Versuch 1 | | | Versuch 2 | | |
| DiPDO:H₂O = 1:4 | | | DiPDO:H₂O = 1:4 | | |
| CH₂SO₄ = 0,5 % | | | CH₂SO₄ = 0,5 % | | |

| t8 [h] | U9 [%] | S10 [%] | t [h] | U [%] | S [%] |
|---|---|---|---|---|---|
| 0,08 | 4,8 | 27,6 | 0,08 | 14,8 | 33,3 |
| 0,16 | 5,8 | 48,6 | 0,16 | 27,2 | 63,1 |
| 0,32 | 16,2 | 35,5 | 0,32 | 47,8 | 60,9 |
| 1 | 33,1 | 58,1 | 1 | 74,8 | 52,8 |
| 2 | 49,5 | 61,4 | | | |

| Versuch 3 | | | Versuch 4 | | |
|---|---|---|---|---|---|
| DiPDO:H₂O = 1:8 | | | DiPDO:H₂O = 1:8 | | |
| CH₂SO₄ = 0,5 % | | | CH₂SO₄ = 0,5 % | | |

| t [h] | U [%] | S [%] | t [h] | U [%] | S [%] |
|---|---|---|---|---|---|
| 0,08 | 2,1 | 34,3 | 0,08 | 4,8 | 80,2 |
| 0,16 | 1,9 | 98 | 0,16 | 14,4 | 74,5 |
| 0,32 | 6,0 | 70,6 | 0,32 | 29,6 | 73,9 |
| 1 | 20,2 | 65,5 | 1 | 64,1 | 68,5 |
| 2 | 35,9 | 68,6 | | | |
| ⁸ Verweilzeit | | | | | |
| ⁹ Umsatz | | | | | |
| ¹⁰ Selektivität | | | | | |

**Tabelle 4:**

| T = 250°C | | | T = 280°C | | |
|---|---|---|---|---|---|
| Versuch 5 | | | Versuch 6 | | |
| DiPDO:H₂O = 1:4 | | | DiPDO:H₂O = 1:4 | | |
| CH₂SO₄ = 2,5 % | | | CH₂SO₄ = 2,5 % | | |

| t [h] | U [%] | S [%] | t [h] | U [%] | S [%] |
|---|---|---|---|---|---|
| 0,08 | 8,7 | 46,8 | 0,08 | 39,6 | 63,6 |
| 0,16 | 21,7 | 50,8 | 0,16 | 68,2 | 54,9 |
| 0,32 | 39,7 | 54,9 | 0,32 | 80,5 | 44,3 |
| 1 | 69,8 | 61,1 | | | |

| Versuch 7 | | | Versuch 8 | | |
|---|---|---|---|---|---|
| DiPDO:H₂O = 1:8 | | | DiPDO:H₂O = 1:8 | | |
| CH₂SO₄ = 2,5 % | | | CH₂SO₄ = 2,5 % | | |

| t [h] | U [%] | S [%] | t [h] | U [%] | S [%] |
|---|---|---|---|---|---|
| 0,08 | 4,8 | 50,5 | 0,08 | 25,2 | 64,3 |
| 0,16 | 11,0 | 65,4 | 0,16 | 51,7 | 69,7 |
| 0,32 | 23,7 | 65,6 | 0,32 | 74,2 | 59,9 |

Aus den acht aufgeführten Versuchen kristallisieren sich die Versuche 2, 4, 5 und 8 heraus. Da es vorteilhafter ist, mit geringen H₂SO₄-Konzentrationen zu arbeiten - in Anbetracht der nachfolgenden Neutralisation der Lösung und Anfall von Salz - werden die Versuchsparameter aus Versuch 4 (mit einer Stunde Verweilzeit) als die optimalen festgelegt.

### 3) Spaltung von im Sumpf der PDO-Reindestillation enthaltenem DiPDO im Tantalrohr

Die PDO-Sumpflösung enthält neben DiPDO noch die beiden cis/trans-Isomere H-HMT1 und H-HMT2 mit ca. 15 % beziehungsweise 7 % in der Organik. Da diese Verbindungen ebenfalls zur PDO-Bildung beitragen, werden diese in die Umsatz- und Selektivitätsberechnungen mit eingezogen.

Es werden verschiedene Versuchsparameter getestet, wobei die H₂SO₄-Konzentration stets 0,5 % bezogen auf die Organik betrug (Tabelle 5).

- Einfluß der Temperatur (Verweilzeit von 0,33 h)

**Tabelle 5:**

| T [°C] | Organik:H2O | DiPDO-Umsatz [%] | PDO-Selektivität [%] |
|---|---|---|---|
| 150 | 1:8 | 0,6 | 23,7 |
| 250 | 1:8 | 4,5 | 22,6 |

Bei der kurzen Verweilzeit von 0,33 h liegen sowohl Umsatz als auch Selektivität relativ niedrig. Temperaturen unter 250°C sind für die DiPDO-Spaltung ungeeignet.

### - Einfluß der DiPDO-Konzentration bei 250°C

Mit den in Tabelle 6 aufgeführten Versuchsparametern werden folgende Umsätze und Selektiväten erzielt:

**Tabelle 6:**

| T [°C] | Organik:H₂O | t [h] | Betriebszeit Reaktor [h] | DiPDO-Umsatz [%] | PDO-Selektivität [%] |
|---|---|---|---|---|---|
| 250 | 1:8 | 2 | 3 | 34,2 | 21,7 |
| | | | 4 | 22,5 | 36,9 |
| 250 | 1:4 | 2 | 3 | 54,7 | 24,8 |
| | | | 4 | 47,0 | 39,3 |
| 250 | 1:1 | 2 | 3 | 62,4 | 30,1 |
| | | | 4 | 61,7 | 30,0 |
| | | | 5 | 60,2 | 29,8 |

Das Organik:H₂O-Verhältnis von 1:1 in der Eduktlösung hat genau wie bei der Rein-DiPDO-Lösung hohe Umsätze, aber relativ niedrige Selektivitäten zur Folge. Die verdünnteren Lösungen liefern steigende Umsätze bei steigenden Organik-Konzentrationen, die Selektivitäten sind nahezu gleich (geringer Anstieg).

### - Ergebnisse der optimalen Versuchsparametern

Mittels den Bedingungen T = 280°C, Organik:H₂O von 1:8, Verweilzeit 1 h und H₂SO₄-Konzentration von 0,5 % erhält man einen Umsatz von 53 % und eine PDO-Selektivität von 44 %.

### 4) Aufarbeitung

### - Neutralisation der Spaltlösung

Die Spaltlösung wird mit wäßriger Ca(OH)₂-Lösung neutralisiert und mit anschließender Filtration vom ausgefallenen CaSO₄ befreit.

### - Destillation

Eine mit Spaltlösung abgemischte PDO-Reaktionslösung wird mittels Restwasserdestillation mit anschließender Zwischensiederdestillation und Reindestillation der erhaltenen Sumpflösungen wie beim herkömmlichen PDO-Verfahren aufgearbeitet. Deszillationsbedingungen sind in Tabelle 7 aufgeführt.

**Tabelle 7:**

| | | | | | |
|---|---|---|---|---|---|
| | Packungen | T [°C] | P [mbar] | Volumenstrom [ml/h] | Rücklaufverhältnis |
| Restwasserdestillation | Sulzer CY 1m, 50 mm | 150 | 100 | 325-250 | 5:1 |
| Zwischensieder-destillation | Sulzer CY 1m, 50 mm | 170 | 50 | 150 | 1:100 |
| Reindestillation | Sulzer CY 1m, 50 mm | 160 | 20 | 230 | 1:1 |

Nachfolgend sind die erhaltenen Produktspektren der bei den Destillationen erhaltenen Lösungen mit denen beim herkömmlichen PDO-Verfahren erhaltenen Lösungen gegenübergestellt.

**Tabelle 8:**

| Spaltlösung (SL), Einsatzlösungen (EL) und Rein-PDO-Lösungen der Destillationen (Zahlenformat xx,xx = GC-Flä.% und xxxx = ppm) | | | | | | | | | |
|---|---|---|---|---|---|---|---|---|---|
| Substanz | SL | EL RWD11 neu | EL RWD alt | EL ZSD12 neu | EL ZSD alt | EL RD13 neu | EL RD alt | Rein-PDO-Lösung neu | Rein-PDO-Lösung alt |
| MeOH14 | 0,17 | 151 | 15 | 108 | 0 | 59 | 35 | 10 | 32 |
| EtOH | 0,13 | 117 | 222 | 0 | 0 | 0 | 0 | 0 | 0 |
| Ac | 0,99 | 436 | 337 | 63 | 18 | 20 | 154 | 26 | 146 |
| AllOH15 | 0,35 | 280 | 0 | 0 | 0 | 0 | 0 | 0 | 0 |
| PrOH | 0,35 | 320 | 157 | 0 | 0 | 0 | 0 | 0 | 0 |
| HPA | 96 | 81 | 84 | 0 | 39 | 0 | 0 | 0 | 0 |
| Et-PDO16 | 290 | 306 | 633 | 0 | 515 | 0 | 0 | 0 | 0 |
| PDO | 34,4 | 82,5 | 86,8 | 84,6 | 87,0 | 79,6 | 86,1 | 99,9 | 99,8 |
| 2M13PED | 153 | 588 | 161 | 338 | 125 | 55 | 76 | 80 | 106 |
| 3-HMT17 | 3,59 | 0,51 | 0,11 | 0,42 | 0,10 | 131 | 117 | 58 | 62 |
| HED18 | 0,27 | 0,34 | 0,53 | 0,29 | 0,53 | 11 | 397 | 49 | 350 |
| 2M15PED 19 | 294 | 0,21 | 0,12 | 0,20 | 0,11 | 0,24 | 0,13 | 0 | 0 |
| cHDO201 | 0 | 0,25 | 0,28 | 0,25 | 0,28 | 0,35 | 0,30 | 0 | 0 |
| cHDO2 | 0 | 0,47 | 0,39 | 0,49 | 0,38 | 0,65 | 0,40 | 0 | 104 |
| DiPDO | 41,9 | 8,84 | 7,45 | 8,75 | 7,51 | 12,4 | 8,65 | 0 | 269 |
| H-HMT1 | 0 | 1,76 | 1,85 | 1,73 | 1,84 | 2,59 | 2,10 | 0 | 417 |
| H-HMT2 | 0 | 0,77 | 0,76 | 0,79 | 0,77 | 1,13 | 0,87 | 17 | 104 |
| 136HT21 | 378 | 0,35 | 0,34 | 0,31 | 0,31 | 0,57 | 0,32 | 0 | 98 |
| TriPDO | 2,10 | 0,18 | 0 | 0,17 | 0 | 0,30 | 0 | 0 | 0 |
| 4-fach-PDO-Ether | 0,35 | 541 | 0 | 371 | 0 | 895 | 0 | 0 | 0 |

| | | | | | | | | | |
|---|---|---|---|---|---|---|---|---|---|
| ¹¹ Restwasserdestillation | | | | | | | | | |
| ¹² Zwischensiederdestillation | | | | | | | | | |
| ¹³ Reindestillation | | | | | | | | | |
| ¹⁴ Methanol | | | | | | | | | |
| ¹⁵ Allylalkohol | | | | | | | | | |
| ¹⁶ 3-Ethoxypropanol | | | | | | | | | |
| ¹⁷ 3-Hydroxymethyltetrahydropyran | | | | | | | | | |
| ¹⁸ 2-(2-Hydroxymethyl)-1,3-dioxan | | | | | | | | | |
| ¹⁹ 2-Methyl-1, 5-pentandiol ²⁰ 1,4-Cyclohexandiol | | | | | | | | | |
| ²¹ 1,3,6-Hexantriol | | | | | | | | | |

Es wird ein Reinprodukt mit 99,9 % PDO erhalten. Die Reinheit des PDO bleibt somit unbeeinflußt trotz Beimischen der Spaltlösung zu Beginn der Destillationen.

## Patentansprüche

1. Verfahren zur Herstellung von 1,3-Propandiol (PDO), umfassend die Stufen
(a) Hydratisierung von Acrolein in Gegenwart eines sauren Hydratisierungskatalysators,
(b) katalytische Hydrierung des 3-Hydroxypropionaldehyd enthaltenden, von nicht umgesetzten Acrolein befreiten Reaktionsgemisches der Stufe (a) und
(c) destillative Aufarbeitung des Wasser, 1,3-Propandiol und oberhalb 1,3-Propandiol siedende Nebenprodukte enthaltenden Reaktionsgemisches der Stufe (b),
**dadurch gekennzeichnet, dass**
man den Hochsiedersumpf der Aufarbeitung (c) - auch als PDO-Sumpflösung bezeichnet - zwecks Etherspaltung in wässriger Lösung in Gegenwart von Säure (n) im Temperaturbereich von 200 bis 300 °C behandelt, die erhaltene Lösung neutralisiert und in die destillative Aufarbeitungsstufe (c) zurückführt.

2. Verfähren gemäß Anspruch 1, worin die Säure eine Mineralsäure oder eine organische Säure ist.

3. Verfahren gemäß Anspruch 1 oder 2, worin die Säure eine Mineralsäure, ausgewählt aus der Gruppe, bestehend aus H₂SO₄, H₃PO₄ und HNO₃, ist.

4. Verfahren gemäß Anspruch 1 oder 2, worin die Säure eine organische Säure, ausgewählt aus der Gruppe, bestehend aus Propionsäure, Trifluoressigsäure und Pyridinhydrochlorid, ist.

5. Verfahren nach einem der Ansprüche 1 bis 4, worin der Hochsiedersumpf weiterhin 4-Hydroxy-3-hydroxymethyltetrahydropyran umfasst.

6. Verfahren nach einem der Ansprüche 1 bis 5, worin die Säure in einer Menge von 0,5 oder 2,5 Gewichtsprozent, basierend auf der Organik, enthalten ist.

7. Verfahren nach einem der Ansprüche 1 bis 6, worin Wasser zum Hochsiedersumpf zugegeben wird, um die wässrige Lösung zu bilden.

8. Verfahren nach Anspruch 7, worin das Wasser zum Hochsiedersumpf in einer Menge zugegeben wird, dass das Verhältnis Organik zu Wasser 1:1, 1:4 oder 1:8 ist.

9. Verfahren nach Anspruch 1 bis 8, wobei dieses kontinuierlich durchgeführt wird.

10. Verfahren nach einem der Ansprüche 1 bis 9, worin
- die verwendete Säure in einer Menge von 0,5 oder 2,5 Gewichtsprozent, basierend auf der Organik, zugegeben wird,
- das Wasser zum Hochsiedersumpf in einer Menge zugegeben wird, dass das Verhältnis Organik zu Wasser 1:1, 1:4 oder 1:8 ist, und
- die Säure eine Mineralsäure ist, ausgewählt aus der Gruppe, bestehend aus H₂SO₄, H₃PO₄ und HNO₃.

## Claims

1. Method for the preparation of 1,3-propanediol (PDO), involving the stages:
(a) hydration of acrolein in the presence of an acid hydration catalyst,
(b) catalytic hydrogenation of the reaction mixture freed of unconverted acrolein and containing 3-hydroxypropionaldehyde from stage (a), and
(c) distillative workup of the reaction mixture of stage (b) containing water, 1,3-propanediol, and by-products boiling above [with boiling points higher than] 1,3-propanediol,
**characterized by** the fact that the high-boiling bottoms from workup (c) - also referred to as PDO bottom solution - are treated for ether cleavage in aqueous solution in the presence of acid(s) in the temperature range of 200 to 300°C, the obtained solution is neutralized, and returned to distillative workup stage (c).

2. Method according to Claim 1, in which the acid is a mineral acid or organic acid.

3. Method according to Claim 1 or 2, in which the acid is a mineral acid chosen from the group consisting of H₂SO₄, H₃PO₄, and HNO₃.

4. Method according to Claim 1 or 2, in which the acid is an organic acid chosen from the group consisting of propionic acid, trifluoroacetic acid, and pyridine hydrochloride.

5. Method according to one of Claims 1 to 4, in which the high-boiling bottoms also contain 4-hydroxy-3-hydroxymethyltetrahydropyran.

6. Method according to one of Claims 1 to 5, in which the acid is contained in an amount of 0.5 or 2.5 wt% in reference to the organic material.

7. Method according to one of Claims 1 to 6, in which water is added to the high-boiling bottoms in order to form the aqueous solution.

8. Method according to Claim 7, in which water is added to the high-boiling bottoms in an amount such that the ratio of organic material to water is 1:1, 1:4, or 1:8.

9. Method according to one of Claims 1 to 8, **characterized by** being run continuously.

10. Method according to one of Claims 1 to 9, in which
- the employed acid is added in an amount of 0.5 or 2.5 wt% in reference to the organic material,
- the water is added to the high-boiling bottoms in an amount such that the ratio of organic material to water is 1:1, 1:4, or 1:8; and
- the acid is a mineral acid chosen from the group consisting of H₂SO₄, H₃PO₄, and HNO₃.

## Revendications

1. La méthode de préparation de 1,3-propanédiol (PDO), impliquant les étapes suivantes :
(a) l'hydratation d'acroléine en présence d'un catalyseur d'hydratation acide,
(b) l'hydrogénation catalytique du mélange réactif dépourvu d'acroléine non convertie et contenant du 3-hydroxypropionaldéhyde issu de l'étape (a), et
(c) le traitement final par distillation du mélange réactif de l'étape (b) contenant de l'eau, du 1,3-propanédiol et des produits dérivés dont le point d'ébullition est supérieur à celui du 1,3-propanédiol,
**caractérisée par le fait que** les queues à point d'ébullition élevé issues du traitement final (c) - aussi appelées « solution de queue PDO » - sont traitées pour le clivage de l'éther en solution aqueuse en présence d'acide(s) dans l'intervalle de températures compris entre 200 et 300°C, la solution obtenue est neutralisée et retournée à l'étape de traitement final par distillation (c).

2. La méthode selon la revendication 1, où l'acide est un acide minéral ou organique.

3. La méthode selon la revendication 1 ou 2, où l'acide est un acide minéral choisi à partir du groupe comprenant H₂SO₄, H₃PO₄ et HNO₃.

4. La méthode selon la revendication 1 ou 2, où l'acide est un acide organique choisi à partir du groupe comprenant l'acide propionique, l'acide trifluoroacétique et l'hydrochlorure de pyridine.

5. La méthode selon la revendication 1 à 4, où les queues à point d'ébullition élevé contiennent également du 4-hydroxy-3-hydroxyméthyltétrahydropyran.

6. La méthode selon l'une des revendications 1 à 5, où l'acide est contenu à raison de 0,5 ou 2,5 pourcentage en masse par rapport à la matière organique.

7. La méthode selon l'une des revendications 1 à 6, où de l'eau est ajoutée aux queues à point d'ébullition élevé pour former la solution aqueuse.

8. La méthode selon la revendication 7, où de l'eau est ajoutée aux queues à point d'ébullition élevé en quantité telle que le rapport matière organique/eau soit de 1:1, 1:4 ou 1:8.

9. La méthode selon l'une des revendications 1 à 8, **caractérisée par** une exécution continue.

10. La méthode selon l'une des revendications 1 à 9, où
- l'acide employé est ajouté à raison de 0,5 ou 2,5 pourcentage en masse par rapport à la matière organique,
- de l'eau est ajoutée aux queues à point d'ébullition élevé en quantité telle que le rapport matière organique/eau soit de 1:1, 1:4 ou 1:8; et
- l'acide est un acide minéral choisi à partir du groupe comprenant H₂SO₄, H₃PO₄ et HNO₃.
